# EUROPEAN PATENT SPECIFICATION

(11) **EP 0 465 441 B1**
(45) Date of publication and mention of the grant of the patent: **22.05.1996**
(21) Application number: 91830296.9
(22) Date of filing: 03.07.1991
(51) Int. Cl.: A61N 1/40, H05B 6/62

(54) **Hyperthermia-inducing apparatus with capacitive rings**
Hyperthermiegerät mit kapazitiven Ringen
Appareil d'hyperthermie à anneaux capacitifs

(30) Priority: 03.07.1990 IT 4810990
(43) Date of publication of application: 08.01.1992
(73) Proprietor: Ente per le nuove tecnologie, l'energia e l'ambiente ( ENEA), I-00198 Roma (IT)
(72) Inventor: Lovisolo, Giorgio Alfonso, I-00184 Rome (IT); Mauro, Francesco, I-00060 Rome (IT); Raganella, Luigi, I-00172 Rome (IT)
(74) Representative: Taliercio, Antonio

(56) References cited:
- DE-C- 959 311
- US-A- 4 296 298
- US-A- 4 374 516
- IEEE TRANSACTIONS ON BIOMEDICAL ENGINEERING vol. 36, no. 11, November 1989, NEW YORK US pages 1124 - 1132; L.RAGANELLA et al.:"27MHz conformal capacitive ring (CR) applicators for uniform hyperthermic/diathermic treatment of body segments with axial fields"

## Description

This invention relates in general to applicators which can be employed for irradiating with radiofrequency electromagnetic radiations some inner zones of the human body, as for instance thorax, abdomen and the upper and lower limbs, in order to obtain hyperthermia in said zones. The working frequency can range from some MHz to some hundred MHz according to the body part that is to be treated. More particularly, this invention relates to an applicator of the type with capacitive rings, which can be employed in the clinical routine for hyperthermic therapy, which is an adjuvant therapy in the treatment of neoplastic diseases.

Hyperthermic therapy is a well known kind of therapy, which was hypotesized and theorized many years ago for the treatment of neoplastic diseases, in particular for the destruction of tumoral cells on the basis of different susceptibilities of normal tissues or of neoplastic tissues to heat.

No known industrial and/or laboratory prototype is able at present to realize a sufficiently efficient heating. Usually, layered manikin of cylindrical or ellipsoidal cross-section, simulating human tissues, are employed for characterizing the prototypes and the appraisal of the applicator performance, and it has been observed that the various applicators already realized and somewhat tested in the laboratory, just privilege a single feature without realizing together all those features which should be obtained harmonically in a single applicator. Indeed, some applicators allow a sufficient increase in temperature (by some degrees) to be realized in the centre of symmetry of the manikin, but they quite often give rise to an excessive increase in temperature in the surface zones and/or in other parts of the body. Other applicators allow a homogeneous heating of the whole irradiated region of the body to be obtained, which region accordingly comprises the tumor, the zone around the tumor and the adjacent surface zones: however in that way they do not realize a selective heating of the tumoral tissue.

A further difficulty is met with when a tumor is to be treated which is not localized at the centre of symmetry of the system, which fact generally occurs in the actual clinical situation. The difficulty consists in that one cannot easily shift the maximum action point from a central position to an out-of-centre position, in addition to the fact that, even though on can suceed in theory, as a matter of practice one is forced to undergo an enormous loss of efficiency. Further and increasing difficulties are met with when manikins are employed which are gradually increasingly similar to reality.

For understanding this invention, it will be useful to set forth in advance a short note on the theory of operation of such applicators as well as on the structures realized up to the present moment or disclosed in the technical literature.

Said applicators generate a discrete or a continuous dipole array around the manikin, such dipoles having a tendency to focus a maximum value of the electric field, and consequently of temperature, at the centre point. However, as already mentioned above, it is not always possible to obtain a maximum focus and the user relies just on the heating of the whole region. This aspect is quite well simulated also by means of theoretical calculation models. It is to be kept in mind that the geometric and the thermal characteristics of the maximum value depend on different parameters: frequency, sizes of the manikin, electrical and thermal properties of the tissues themselves. Moreover, in the in vivo configurations, they depend on the inner biological systems as well as on their operation. The structures which are most employed at the present time for generating this set of dipoles are: a) two capacitive rings, b) two capacitive rings divided into four portions, c) a number (n) of waveguides, and d) n dipoles. The last two members are the most studied systems at present.

The technical possibilities for shifting the maximum to a non-central zone are strictly connected to the technique employed for heating the body part; indeed, such shift can be obtained by a) shifting the manikin inside the rings, b) changing, at the same time or not, two parameters i.e. the phase and the strength on each of the four sections of the ring, c) changing, simultaneously or not, two parameters, i.e. the phase and the strength, within the n waveguides or within said n dipoles.

Document IEEE Transactions on Biomedical Engineering, Vol. 36, No. 11, Nov. 1989, 1124-1132 discloses a capacitive ring hyperthermia applicator comprising a pair of capacitive rings interconnected to each other by means of a line C, L₂, L₁ and feeding means RF for feeding at a radio frequency said pair of capacitive rings.

As the industrial products available at present realize a hyperthermic heating which is not fully efficient and controlled, as was already stressed above, it is the specific object of this invention to povide a structure which, in the evolution of the studies carried out up to the present time, does not give the drawbacks and the practical operative difficulties occurring in the realizations already known.

This object is obtained by means of a hyperthermia applicator of the above kind, wherein in addition to said first pair of capacitive rings being interconnected by at least one lines, it comprises a second pair of capacitive rings which are also interconnected by means of at least one lines, each of the at least one lines interconnecting the first pair of capacitive rings being adjustably and inductively coupled to a respective one of the at least one lines interconnecting the second pair of capacitive rings, the second pair of capacitive rings working in a different phase with respect of the first pair and being coupled to them inductively; whereby said feeding means feed at least one of said two pairs of capacitive rings.

Further details and advantages of this invention will be evident from the following disclosure with reference to the enclosed drawings, wherein the preferred embodiment of the invention is shown just for illustrative and not for limitative purposes.

In the drawings:
Figure 1 shows a schematic view of an applicator according to the present invention, in a scheme in which a direct feeding is provided;
Figure 2 shows a schematic view of an applicator according to this invention, in a scheme wherein a magnetic coil feeding is provided;
Figures 3, 4 and 5 show three energy release diagrams (the temperature difference is shown on the ordinate axis) in three different kinds of employment of an applicator according to this invention.

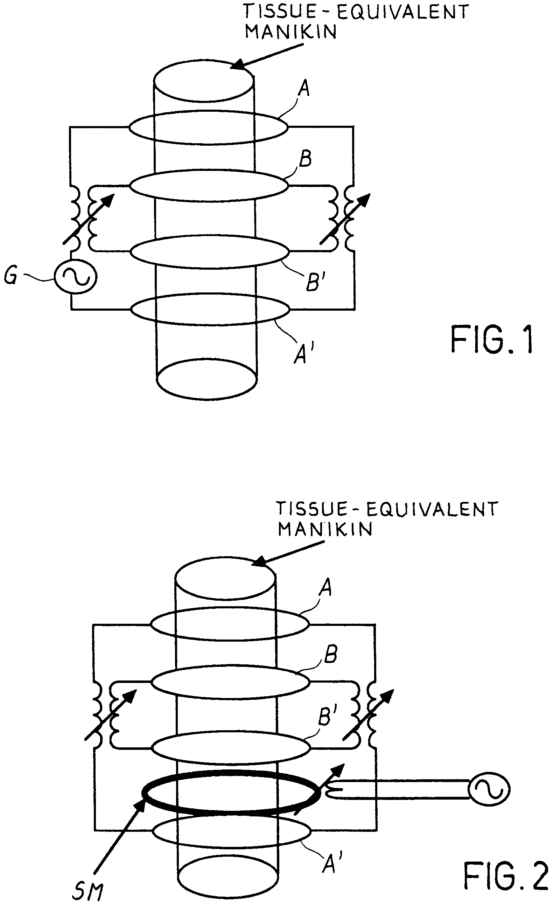

With reference now to Figures 1 and 2, it can be observed that the applicator according to the present invention comprises two pairs of rings: an active pair A-A' and a passive pair B-B', 180° out of phase and inductively coupled to the first pair. The passive ring pair B-B' can be divided into n parts along the circumference and each one of said parts has an independent inductive coupling.

The whole set gives rise to a field - which can be considered as made up of two fields - which is completely different from the filed generated in any possible configuration by a single pair of rings. In a more detailed way, it can be observed that the two pairs of rings are arranged along the z axis so as to encircle the body to be irradiated; the rings are connected two by two to one or more connection lines.

No operation limits have been put into evidence as regards the sizes of the rings: just for exemplification purposes it is reported that rings whose sizes along the z-axis were between 1 and 6 cm have been tested successfully.

No limits have also been put into evidence as regards the wavelength range to be employed, as frequencies between 27 and 144 MHz have been tested successfully, said values also being reported just for exemplification purposes.

As far as the way of feeding the applicator, it can be set forth that the applicator can be fed directly as shown in Figure 1, by means of a generator G of the desired frequency, or by means of a magnetic turn SM arranged between the body to be treated and the ring connection lines. The well known TORO system (with a retractable resonant cavity) is an example of the latter way of feeding the applicator.

As already mentioned above, the outer ring pair A-A′ is the active pair, whereas the inner B-B′ pair is the passive pair. According to a preferred ambodiment of the invention, the rings of the passive pair are divided into a number of sections which are interconnected by means of a corresponding line. The divisions along the passive rings and the corresponding independent coupling allow the strength of the electric field to be varied in some zones, so modifying the total field with the possibility of obtaining the formation of the maximum value in a zone out of the centre.

According to a different kind of embodiment, both the ring pairs are fed directly, so transforming both pairs into active pairs. Such solution could allow the relative phases to be changed so as to employ for optimization purposes also the possibilities offered by the phase change.

Further according to a different kind of embodiment, both the ring pairs are divided into sections, and in that case also the feeding can be limited to a pair only or it can be applied to both ring pairs so sectioned.

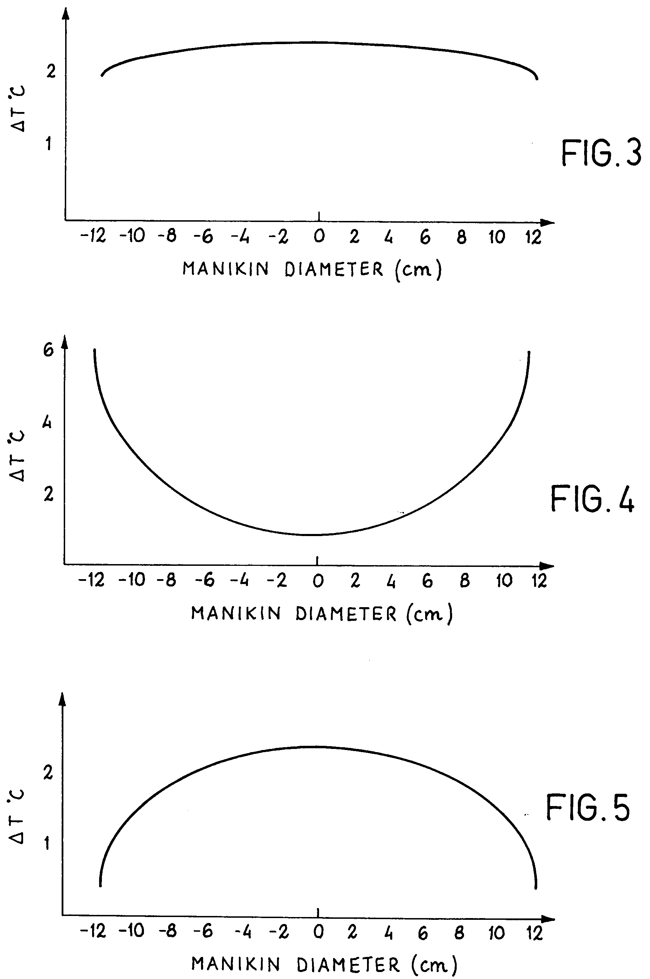

If one supposes to feed just the active pair A-A′ as shown in Figure 1, the operation of the applicator, working at one of the frequencies selected for heating thorax, is as follows:
the active pair A-A′ generates an electric field with a small maximum at the centre as shown in Figure 3, which shows the release of energy with a ring pair arranged at 30 cm distance along the z-axis, at a power of about 200 W and at 27 MHz. The passive pair B-B′, having unsectioned rings and being is energized inductively by the active pair, generates a very strong electric field on the outer surface of the body to be heated (the manikin) as shown in Figure 4, which shows the release of energy with a pair of rings arranged at 6 cm distance along the z-axis, at a power of about 200 W and at 27 MHz. The two fields so generated overlap and result in the optimization of the maximum value at the centre, as shown in Figure 5. The latter thus shows the release of energy with two pairs of rings at 30 cm and at 6 cm along the z-axis, at a power of about 200 W and a frequency of 27 MHz.

In order to increase further the possibilities of employment and then to improve on the flexibility of the application the rings can be realized also with remarkably larger diameters with respect to the size of the body to be treated, so that the body itself can be shifted inside the rings so as to cause the point of maximum value to coincide with the centre of the tumor.

Finally, although rings and diameters have been expressly mentioned, it is to be clearly set forth that it is not intended to limit the shape of the rings to the circular shape exclusively, because the principles and the operating ways of the invention hold good also for rings of elliptical or oval shapes or other similar shapes.

The preferred embodiments have been disclosed in the preceding text, but it is to be undderstood that those who are skilled in the art can introduce further modifications and changes without departing from the scope of the present invention as defined in the appended claims.

## Claims

1. A capacitive ring hyperthermia applicator comprising a pair of capacitive rings interconnected to each other by means of one line and feeding means for feeding at radio frequency said pair of capacitive rings
characterized in that,
in addition to said first pair of capacitive rings being interconnected by at least one lines, it comprises a second pair of capacitive rings which are also interconnected by means of at least one lines, each of the at least one lines interconnecting the first pair of capacitive rings being adjustably and inductively coupled to a respective one of the at least one lines interconnecting the second pair of capacitive rings, the second pair of capacitive rings working in a different phase with respect of the first pair and being coupled to them inductively; whereby said feeding means feed at least one of said two pairs of capacitive rings.

2. An applicator according to claim 1, characterized in that the rings of at least said second pair are divided into n sections which are interconnected respectively with one or more lines.

3. An applicator according to claims 1 or 2, characterized in that said feeding means are made of a radiofrequency generator that feeds directly said first pair of capacitive rings.

4. An applicator according to claim 3, characterized in that said radiofrequency is in the range between some MHz and some hundreds MHz, and in particular is between 27 and 144 MHz.

5. An applicator according to claims 1 or 2, characterized in that said feeding means are made up of a magnetic turn interposed between the body to be heated and the connection lines between the rings.

6. An applicator according to claim 5, characterized in that said magnetic turn is of the retractable resonant cavity type.

7. An applicator according to claims 1-6, characterized in that the rings of both said pairs of capacitive rings are divided into sections.

8. An applicator according to claims 1-7, characterized in that both said pairs of capacitive rings are fed.

9. An applicator according to claims 1-8, characterized in that said rings can be of a shape selected from the circular, the elliptical, the oval shape or similar shapes.

10. An applicator according to claims 1-9, characterized in that said rings are of 1-6 cm height.

## Patentansprüche

1. Hyperthermiegerät mit kapazitiven Ringen, mit einem Paar von kapazitiven, miteinander durch eine Leitung und Speisemitteln verbundenen Ringen, zum Speisen des vorgenannten Paars von kapazitiven Ringen bei Rundfunkfrequenz, dadurch gekennzeichnet, dass zusätzlich zum vorgenannten ersten Paar von kapazitiven, durch eine erste Leitung miteinander verbundenen Ringen, ein zweites Paar von kapazitiven, gleichfalls durch mindestens eine Leitung miteinander verbundenen Ringen vorgesehen ist, wobei je eine der mindestens einer Leitung, die die beiden Ringe des ersten Paars miteinander verbindet, mit der entsprechenden mindestens einer, die beiden Ringe des zweiten Ringpaares miteinander verbindenden Leitung einstellbar und induktiv verbunden ist und wobei das zweite Paar von kapazitiven Ringen in einer zum ersten Ringpaar unterschiedlichen Phase arbeitet und mit demselben induktiv gekuppelt ist, wodurch die vorgenannten Speisemittel mindestens das eine der genannten beiden Paare von kapazitiven Ringen speisen.

2. Hyperthermiegerät nach Anspruch 1, dadurch gekennzeichnet, dass die Ringe des vorgenannten mindestens einen zweiten Paars in n Ausschnitte unterteilt sind, die miteinander durch eine beziehungsweise mehrere Leitungen verbunden sind.

3. Hyperthermiegerät nach Anspruch 1 oder 2, dadurch gekennzeichnet, dass die vorgenannten Speisemittel aus einem Radiofrequenzgenerator bestehen, der das vorgenannte erste Paar von kapazitiven Ringen unmittelbar speist.

4. Hyperthermiegerät nach Ansprueche 3, dadurch gekennzeichnet, dass die Radiofrequenz im Bereich von einigen MHz bis einigen hunderten MHz, insbesondere zwischen 27 und 144 Mhz liegt.

5. Hyperthermiegerät nach Anspruch 3, dadurch gekennzeichnet, dass die vorgenannten Speisemittel aus einer magnetischen, zwischen dem zu erwärmenden Körper und Verbindungsleitungen der Ringe angeordneten Windung bestehen.

6. Hyperthermiegerät nach Anspruch 4 dadurch gekennzeichnet, dass die vorgenannte magnetische Windung nach Art eines rückziehbaren Resonanzhohlraumes ausgebildet ist.

7. Hyperthermiegerät nach Anspruechen 1 bis 6, dadurch gekennzeichnet, dass die Ringe der beiden vorgenannten Kapazitivringepaaren in Ausschnitte unterteilt sind.

8. Hyperthermiegerät nach Ansprueche 1 bis 7, dadurch gekennzeichnet, dass die beiden Paare von Kapazitivringen gespeist sind.

9. Hyperthermiegerät nach Ansprueche 1 bis 8, dadurch gekennzeichnet, dass die vorgenannten Ringe kreisförmig, elliptisch, einförmig oder ähnlich ausgebilet sein können.

10. Hyperthermiegerät nach Ansprüche 1 bis 9, dadurch gekennzeichnet, dass die vorgenannten Ringe eine Höhe 1 bis 6 cm haben.

## Revendications

1. Appareil d'hyperthermie à anneaux capacitifs comprenant un couple des anneaux capacitifs joints entre eaux par une ligne et moyeans d'alimentation à radiofréquence pour allimenter ledit couple des anneaux capacitifs, caractérisé en ce que outre ledit couple des anneaux capacitifs, assemblés entre eux par au moin une ligne, il comprend un deuxième couple des anneaux capacitifs, qui sont aussi joint entre eux par au moins une ligne, chacunne des lesdits au moin une ligne, qui assemble entre eux la premiere couple des anneaux capacitifs, étant réglable et accouplé inductivement à la ligne réspective des au moins une ligne, qui assemble entre eau ledit deuxième couple des anneaux, le deuxième couple des anneaux capacitifs travaillant dans une phase différente par rapport à le premiere couple et étant accouplé inductivement à eux, raison pour laquelle lesdits moyens d'alimentation alimentent au moins un desdits deux couple des anneaux capacitifs.

2. Appareil selon la revendication 1, caractérisé en ce que au moins les anneaux dedit deuxième couple sont dividés en n sections, qui sont assemblés respectivement avec une ou plus lignes.

3. Appareil selon la revendication 1 ou 2, caractérisé en ce que lesdits moyens d'alimentation consistent d'un générateur de radiofréquence, qui alimente directement ledit premier couple des anneaux capacitifs.

4. Appareil selon la revendication 3, caractérisé en ce que ladite radiofréquence se trouve dans un champ entre quelques MHz et quelques centains de Mhz, notamment entre 27 and 144 MHz.

5. Appareil selon la revendication 1 ou 2, caractérisé en ce que lesdit moyens d'alimentation sont faits d'une spire magnétique placée entre le corps à chauffer et le ligne de connexion entre les anneaux.

6. Appareil selon la revendication 5, caractérisé en ce que ladite spire magnétique est du type de cavité de résonance rétractable.

7. Appareil selon les revendications 1-6, caractérisé en ce que les anneaux des deux couples des anneaux capacitifs sont subdivisés en sections.

8. Appareil selon les revendications 1-7, caractérisé en ce que lesdites deux couples des anneaux capacitifs sont alimentés.

9. Appareil selon les revendications 1-8, caractérisé en ce que lesdits anneaux peuvent avoir une forme choisie entre les formes circulaire, elliptique, ovale ou similaire.

10. Appareil selon les revendications 1-9, caractérisé en ce que lesdits anneaux out un hauteur de 1-6 cm.
